Europäisches Patentamt

European Patent Office

Office européen des brevets

(11) Veröffentlichungsnummer: **0 259 411 B1**

# (12) EUROPÄISCHE PATENTSCHRIFT

(45) Veröffentlichungstag der Patentschrift: **12.08.92**

(21) Anmeldenummer: **87901472.8**

(22) Anmeldetag: **03.03.87**

(86) Internationale Anmeldenummer:
**PCT/EP87/00125**

(87) Internationale Veröffentlichungsnummer:
**WO 87/05322 (11.09.87 87/20)**

(51) Int. Cl.⁵: **C12M 1/12**

(54) **VERFAHREN UND VORRICHTUNG ZUM BEHANDELN EINER BIOKATALYSATOREN ODER VERGLEICHBARE TEILCHEN ENTHALTENDEN FLÜSSIGEN MISCHUNG.**

(30) Priorität: **06.03.86 AT 576/86**

(43) Veröffentlichungstag der Anmeldung:
**16.03.88 Patentblatt 88/11**

(45) Bekanntmachung des Hinweises auf die
Patenterteilung:
**12.08.92 Patentblatt 92/33**

(84) Benannte Vertragsstaaten:
**AT BE CH DE FR GB IT LI LU NL SE**

(56) Entgegenhaltungen:
**EP-A- 0 086 539      WO-A-86/07284
CH-A- 497 910      DE-A- 1 436 287
DE-A- 1 461 510      DE-A- 2 146 838
US-A- 326 817      US-A- 4 251 633**

(73) Patentinhaber: **Chemap AG
Hölzliwisenstrasse 5
CH-8604 Volketswil(CH)**

(72) Erfinder: **KATINGER, Hermann, Wolf-Dietrich
Peter-Jordan-Str. 82
A-1100 Wien(AT)**

(74) Vertreter: **Meyer, Ludgerus
Patentanwälte Meyer, Stach, Vonnemann
Jungfernstieg 38
W-2000 Hamburg 36(DE)**

EP 0 259 411 B1

## Beschreibung

Die Erfindung betrifft ein Verfahren zum Behandeln einer suspendierte biologisch aktive Teilchen, wie beispielsweise Biokatalysatoren, Zellkulturen, Mikroorganismen, Mikrocarrier, enthaltenden flüssigen Mischung, bei welchem man diese Flüssigkeit unter Zurückhaltung von Teilchen vorbestimmter Mindestgröße durch eine in Bewegung versetzte Siebfläche hindurchtreten läßt.

Es ist bekannt, Biokatalysatoren, wie Mikroorganismen, Zellkulturen, Carrier mit oder ohne gebundene, biologisch aktive Substanzen, etc. durch mechanische Mittel, wie Siebe mit relativ zur Teilchengröße der zurückzuhaltenden Teilchen engerer Durchgangsweite der Öffnungen, oder durch Anhaften der Biokatalysatoren an Einbauten bzw. statischen oder in der Flüssigkeit suspendierten Trägerteilchen, oder durch Einschließen der Zellen in Gele, wie z.B. Alginate oder dergleichen, oder auch durch Sedimentation gegen ein Ausschwemmen zu immobilisieren. Alle angeführten Methoden haben in der Praxis jedoch erhebliche Nachteile. So neigen Siehe stark zum Verlegen, da die Teilchen durch die Flüssigkeitsströmungen in die Durchgangsöffnungen des Siebes hineingedrängt werden, diese daher von Zeit zu Zeit freigelegt und die Teilchen wieder in Suspension gebracht werden müssen. Auch ist ein Aufheben der Rückhaltewirkung nur durch spezielle Maßnahmen, wie Entfernen des Siebes oder Ausschwemmen mit hoher Durchsatzrate erreichbar. Bei an Füllkörpern oder Einbauten anhaftenden Biokatalysatoren ist die Kultur sehr sensibel, da vielfach ein von den Idealbedingungen abweichender Betrieb ein Abfallen der Biokatalysatoren bewirken kann. Außerdem ist der für das Anhaften benötigte Teil der Biokatalysatoren für die Reaktion blockiert. Ähnliches gilt auch für in Gele oder dergleichen eingeschlossene Biokatalysatoren, bei welchen Diffusionsbarrieren entstehen und biologisch nicht mehr wirksame Partikel noch als Ballast im Reaktionsraum verbleiben. Die Biokatalysatoren werden daher vielfach als solche im Reaktionsgefäß in Suspension gehalten, wobei die im Zuge der Gewinnung des Reaktionsproduktes aus dem Reaktionsgefäß ausgeschwemmten Partikel mechanisch, beispielsweise durch Separation, Sedimentation oder dergleichen aus der Flüssigkeit abgetrennt werden.

Aus der CH-A-602 162 ist ein Verfahren und eine Vorrichtung zum Klassieren von in Flüssigkeiten suspendierten Feststoffen bekannt, bei welchen die Mischung einem in Schwingungen versetzten Siebkorb zugeführt wird, durch den die Flüssigkeit und die erwünschten kleinen Partikel hindurchtreten, während die nicht durch die Sieböffnung passenden Teilchen zurückgehalten werden, aber die Vibration den Aufbau eines Filterkuchens verhindert. Diese Arbeitsweise betrifft jedoch nicht das Einleiten von Gasen oder Nährstoffen in die Flüssigkeit.

Aus der EP-A-86539 ist ein Verfahren und eine Vorrichtung zum Züchten von Mikroorganismen unter gleichzeitiger Abtrennung und Entfernung der entstandenen Stoffwechselprodukte mittels Ultrafiltration bekannt. Dabei wird die mit Mikroorganismen angereicherte Flüssigkeit im Fermenter mit einer Rührvorrichtung durchmischt. Die Rührvorrichtung umgibt dabei den zylinderförmigen Membranfilter, so daß durch die Rotation der Rührvorrichtung mit den daran befestigten Schikanen in unmittelbarer Nähe der Membran eine so starke Turbulenz erzeugt wird, daß die Membran frei von Ablagerungen bleibt. Als Variante kann auch der Membranfilter selbstrotieren, wobei dann der Schikanenkorb feststeht. Nachteilig ist, daß das Membranfilter sehr kleine Öffnungen aufweist, die trotz der turbulenten Umströmung relativ leicht verstopfen. Ferner treten zwischen den mit der Rührvorrichtung rotierenden Schikanen und dem Membranfilter starke Scherungskräfte auf, die die Mikroorganismen beschädigen können. Außerdem ist die Rückhaltewirkung des Membranfilters nicht regelbar, so daß schon für kleinere Anpassungen der Filterwirkung das Membranfilter gewechselt werden muß.

Aus der nachveröffentlichten WO-A-86/07284 ist ein Filter und ein Filterverfahren zur Filterung von Mikroorganismen bzw. Makromolekülen bekannt, bei dem der Filterkörper während des Filterprozesses schwingt. Dabei ist die Filterwirkung einstellbar, so daß bestimmte Teilchen zurückgehalten bzw. durchgelassen werden. Im stationären Zustand ist das Filter durchlässig. Das Filter besteht aus einem porösen piezoelektrischen Material an das eine elektrische Wechselspannung gewünschter Amplitude und Frequenz angelegt wird. Aus der DE-A-2146838 ist ein Verfahren zur Filtration bekannt, bei dem ein Filtermedium mit für die zurückzuhaltenden Teilchen in Ruhe zustand passierbaren Öffnungen Verwendet wird. Die zu filtrierende Suspension wird in zur Filterfläche parallele Schwingungen versetzt, wodurch Teilchen zurückgehalten werden, welche das ruhende Filtermedium passieren würden.

In der US-A-4251633 wird ein Verfahren zum Behandeln von Zellkulturen beschrieben, bei dem der Flüssigkeitsdurchtritt durch eine bewegte Membran erfolgt. Begast wird die Flüssigkeit zu beiden Seiten der Membran.

Aufgabe der Erfindung ist es, ausgehend von der EP-A-86 539 ein Verfahren und eine Vorrichtung zum Behandeln einer flüssigen, biologisch aktiven Mischung zu schaffen, die auf einfache Weise eine weitestgehende, leicht regel- und aufhebbare Rückhaltung von suspendierten biologisch aktiven Teilchen, wie beispielsweise Biokatalysatoren, Zellkulturen, Nährstoffe, Mikroorganismen, Mikrocarriern, ermöglicht und Verstopfungen der Siebflächen vermeidet, und die ferner eine weitgehend blasenfreie Begasung ermöglicht.

Zur Lösung dieser Aufgabe weist das Verfahren die in Kennzeichen von Anspruch 1 genannten Merkmale auf.

Durch die Schwingungen der Siebfläche werden in der flüssigen Mischung Druckwellen erzeugt, die einen Durchtritt suspendierter biologisch aktiver Teilchen, wie beispielsweise Biokatalysatoren, Zellkulturen, Mikroorganismen, Mikrocarriern, durch die demgegenüber größeren Sieböffnungen weitestgehend verhindert und gleichzeitig auch ein Verstopfen der Öffnungen durch Brückenbildung vermeidet. In kleine Fragmente zerfallene, biologisch inaktive Partikel können dagegen durch die Sieböffnungen hindurchtreten. Zur Aufhebung der Rückhaltewirkung genügt es, die Vibration der Siebfläche zu beenden bzw. deren Amplitude und/oder Frequenz entsprechend zu verändern.

Vorteilhafte weitere Ausgestaltungen des Verfahrens sind in den Unteransprüchen 2 bis 4 beschrieben.

Gegenstand der Erfindung ist ferner eine Vorrichtung zum Behandeln einer flüssigen Mischung der eingangs genannten Art, die mit den Merkmalen des Patentanspruchs 5 ausgestattet ist.

Diese Vorrichtung gestattet bei einfacher Konstruktion durch entsprechende Wahl der durch die Vibrationsvorrichtung erzeugten Schwingungen die Rückhaltewirkung auf den jeweils gewünschten Wert einzustellen und die Frequenz und/oder die Amplitude der Schwingungen auch während des Betriebes jederzeit zweckentsprechend zu verändern. Da Biokatalysatoren im Gegensatz zu Füllstoff- oder Trägerpartikeln vielfach begrenzt deformierbar sind, können durch zweckentsprechende Abstimmung der Frequenz und der Amplitude der Schwingungen unter Berücksichtigung der Durchtrittsgeschwindigkeit der Flüssigkeit durch die Sieböffnungen spezielle Rückhalteeffekte erreicht werden. Da bei der erfindungsgemäßen Vorrichtung keine drehenden Teile vorhanden sind, ist ein Dauerbetrieb unter sterilen Bedingungen leicht möglich. Ferner kann die vom Siebkörper umschlossene Flüssigkeitssäule wirksam mit Gas angereichert werden. Durch die vertikale Vibration des Siebkörpers werden in der dessen Innenraum ausfüllenden Flüssigkeit entsprechende Strömungen erzeugt, die eine Verteilung des Gases in der Flüssigkeit fördern, so daß eine weitgehend blasenfreie Begasung erreicht wird. Dies ist ein wichtiger Vorteil, da Gasbläschen für manche Reaktionen schädlich sind, weil es zu Fehlreaktionen im Schaumraum kommen kann. Die im Innenraum des vibrierenden Siebkörpers mit gelöstem Gas gesättigte Flüssigkeit kann durch den Siebkörper nach außen in den die zurückzuhaltenden Biokatalysatoren enthaltenden Reaktionsraum diffundieren, so daß dort keine Gasblasen auftreten und sich eine aufwendige Permeationsbegasung durch im Reaktionsraum verlegte Permeationsschläuche erübrigt.

Vorteilhafte weitere Ausgestaltungen der Vorrichtung sind in den Unteransprüchen 6 bis 12 beschrieben.

Um eine besonders gute Gassättigung im Innenraum des zylindrischen Siebkörpers zu erzielen, kann dieser mit einem oder mehreren an sich bekannten Lochscheiben verbunden sein, die jeweils eine Mehrzahl von sich nach oben oder nach unten stetig verengenden Durchbrechungen aufweisen. Solche Lochscheiben oder ähnliche Mischeinrichtungen können zweckmäßig auch über den Siebkörper radial auswärts vorstehen und auch in diesem Bereich mit entsprechenden Durchbrechungen versehen sein, was eine gute Durchmischung der durch den Siebkörper in den Reaktionsraum eindiffundierenden, mit Gas gesättigten Flüssigkeit mit der dort befindlichen Mischung gewährleistet und außerdem dazu beiträgt, ein Verschließen der Sieböffnungen durch die zurückzuhaltenden Teilchen zu verhindern. Da die Flüssigkeit im wesentlichen nicht kompressibel ist, tritt bei der Vibration der mit dem Siebkörper verbundenen Lochscheiben infolge der konischen Ausbildung der Durchbrechungen eine Beschleunigung der durch diese hindurchgepreßten Flüssigkeitsströme ein, die zu einer intensiven Durchmischung und einem erhöhten interphasialen Austausch zwischen Gas und Flüssigkeit führt, so daß das aus dem Gaseinleitrohr austretende Gas rasch gelöst und eine Sättigung der Flüssigkeit mit gelöstem Gas erzielt wird. Wenn in den Lochscheiben in der Nähe der Siebfläche noch zusätzliche Rückströmöffnungen angebracht sind, werden diese Wirkungen noch verbessert.

Um die Vorrichtung auch mit wechselndem Flüssigkeitsstand betreiben zu können, kann das Gaseinleitrohr mittels geeigneter Umschaltventile auch alternativ zum Einleiten von Gas und zum Abziehen von Flüssigkeit benutzt werden.

Im folgenden wird eine bevorzugte Ausführungsform der erfindungsgemäßen Vorrichtung an Hand der beigefügten Zeichnungen weiter erläutert. Es zeigen:

Fig. 1      einen schematischen Vertikalschnitt durch die Vorrichtung und

Fig. 2      einen schematischen Querschnitt einer den Siebkörper mit dem Gaseinleitrohr verbindenden Lochscheibe.

Fig. 3      eine graphische Darstellung der bei einer derartigen Vorrichtung erzielten Belüftung und

Fig. 4      eine graphische Darstellung der Eregebnisse einer Kultivierung von Säugetierzellen auf Microcanien.

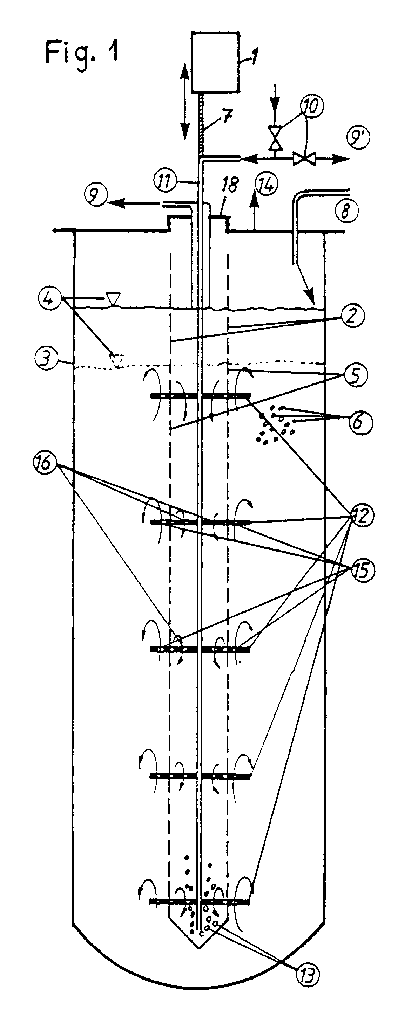

Die in Fig. 1 schematisch dargestellte Vorrichtung umfaßt einen durch einen Deckel dicht verschlossenen, im wesentlichen zylindrischen Behälter 3 mit gewölbtem Boden und eine durch eine zentrische Öffnung des Deckels koaxial in den Behälter 3 bis nahe an dessen Boden hineinragende Rückhaltevorrichtung 2 mit einem zur Wandung des Behälters 3 konzentrischen, zylindrischen Siebkörper 5, der durch mehrere Lochscheiben 12 mit einem zentrischen Gaseinleitrohr 11 zu gemeinsamer Bewegung verbunden ist. Das vertikale Gaseinleitrohr 11 ist über einen Schaft 7 mit einer regelbaren Vibrationsvorrichtung 1 verbunden, mittels deren die gesamte Rückhaltevorrichtung 2 über den Schaft 7 und das Gaseinleitrohr 11 in Schwingungen versetzt werden kann, deren Amplitude und/oder Frequenz an der Vibrationsvorrichtung 1 einstellbar ist. Der den Deckel durchsetzende Teil des Gaseinleitrohres 11 ist von einem Ablaufrohr 9 umgeben, dessen unteres Ende auf der Höhe des maximalen Füllniveaus 4 des Behälters 3 endet. Der Ringraum zwischen dem Ablaufrohr 9 und dem Umfangsrand der zentrischen Öffnung des Deckels ist durch eine Schwingmembran 18 hermetisch verschlossen.

Der Behälter 3 ist bis zur Höhe des Füllniveaus 4 mit einer flüssigen Mischung gefüllt, die Biokatalysatoren 6 suspendiert enthält. Über ein im Deckel angeordnetes, absperrbares Zulaufrohr 8 kann weitere flüssige Mischung oder für die beabsichtigten biologischen Reaktionen erforderliche Hilfsstoffe, beispielsweise ein Nährmedium zugeführt werden. Der Siebkörper 5 beisitzt eine Vielzahl gleichmäßiger Sieböffnungen, deren freie Durchgangsweite hinreichend größer bemessen ist, als die Teilchengröße der Biokatalysator-Teilchen 6. Bei stillgesetztem Siebkörper 5 können diese Teilchen daher durch die Sieböffnungen hindurchtreten. Im Betrieb wird der Siebkörper 5 jedoch durch die Vibrationsvorrichtung 1 in Schwingungen versetzt, deren Frequenz und Amplitude unter Berücksichtigung der Druchtrittsgeschwindigkeit der Flüssigkeit durch die Sieböffnungen so aufeinander abgestimmt sind, daß die zurückzuhaltenden Biokatalysator-Teilchen 6 im wesentlichen vollständig an einem Hindurchtreten durch die Sieböffnungen gehindert werden. Im Betrieb wird ferner zweckmäßig über ein Umschaltventil 10 und das Gaseinleitrohr 11 ein geeignetes Gas oder Gasgemisch in den untersten Teil der in der Rückhaltevorrichtung 2 vom zylindrischen Siebkörper 5 umschlossenen Flüssigkeitsäule eingeleitet. Durch die Vibration des Siebkörpers 5 und der Lochscheiben 12 werden die aus dem unteren Ende des Gaseinleitrohres 11 austretenden, feinen Gasbläschen in der umgebenden, intensiv durchmischten Flüssigkeit mit immer neuer aufnahmebereiter Flüssigkeits in Berührung gebracht, so daß die für die beabsichtigte Reaktion benötigten Gasbestandteile in der umgebenden Flüssigkeit gelöst werden. Da die Gasbläschen 13 durch die nur etwa 10 bis 100 $\mu$m großen Sieböffnungen des Siebkörpers 5 nicht hindurchtreten können, andererseits aber die mit den gelösten Gasbestandteilen angereicherte oder gesättigte Flüssigkeit ohne weiteres durch die Sieböffnungen hindurchdiffundieren kann, wird durch die Vibration der Rückhaltevorrichtung 1 und die damit verbundene Pumpwirkung auch in dem den zylindrischen Siebkörper 5 umgebenden Reaktionsraum eine rasche und gute Verteilung der mit den Gasbestandteilen angereicherten Flüssigkeit erreicht. Da diese gleichzeitig auch mit der über das Zulaufrohr 8 zugeführten Flüssigkeit vermischt wird, ergeben sich bei im wesentlichen vollständiger Rückhaltung der Biokatalysator-Teilchen 6 sehr günstige Reaktionsbedingungen für deren Kultivierung bzw. die angestrebten Reaktionen.

Wenn der Behälter 3 bis zum Füllniveau 4 befüllt ist, kann zur Vermeidung eines weiteren Anstieges des Füllniveaus überschüssige Flüssigkeit über die Ablaufleitung 9 abgezogen werden. Soweit bei der im Behälter 3 durchgeführten Kultivierung in der Flüssigkeit lösliche oder durch die Sieböffnungen des Siebkörpers 5 abfließende Wertsubstanzen gebildet werden, können diese zusammen mit der Flüssigkeit über die Ablaufleitung 9 abgeführt und gewonnen werden. Bei einem Betrieb der Vorrichtung mit nur teilweiser Befüllung oder wechselndem Füllniveau kann die mit Wertsubstanzen angereicherte Flüssigkeit auch über das Gaseinleitrohr 11 und ein Umschaltventil 10 in eine weitere Ablaufleitung 9' abgeführt werden.

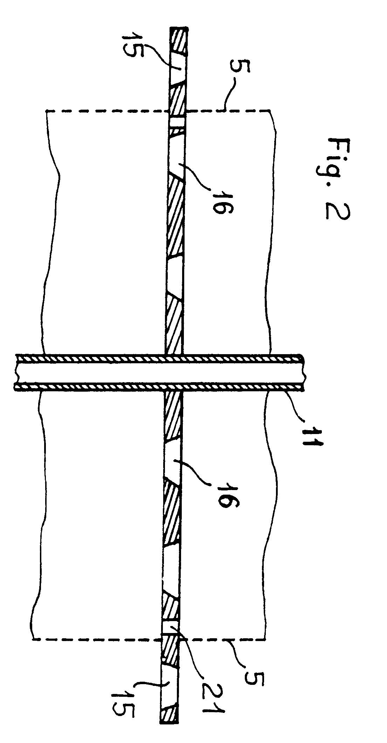

Die in Fig. 2 schematisch dargestellte Lochscheibe 12 ist einerseits mit dem zentrischen Gaseinleitrohr 11 und andererseits mit dem Siebkörper 5 zu gemeinsamer Bewegung verbunden und weist in ihrem vom Siebkörper 5 umschlossenen Bereich eine Vielzahl von sich nach unten konisch verengenden Durchgangsöffnungen 16 sowie nahe der Siebfläche zusätzliche Rückströmöffnungen 21 auf. In dem über dem Siebkörper 5 radial auswärts vorstehenden Außenrand der Lochscheiben 12 sind ferner sich nach oben konisch verengende Durchgangsöffnungen 15 angebracht. Wenn die Rückhaltevorrichtung 2 mittels der Vibrationsvorrichtung 1 in vertikale Schwingungen versetzt wird, erzeugen die konischen Durchgangsöffnungen 15 und 16 durch die Pfeile in Fig. 1 angedeutete Flüssigkeitsströme mit entsprechendem Pumpwirkung. Anstelle der in Fig. 2 dargestellten Lochscheiben 12 können auch Scheiben mit sich in anderer Weise nach unten bzw. nach oben stetig verkleinerndem Öffnungsquerschnitt oder andere Mischelemente zur Erzeugung von die Durchmischung fördernden Flüssigkeitsströmen verwendet werden. Durch die Pumpwirkung der Durchgangsöffnungen 16 werden die Gasbläschen 13 ständig im Innenraum des Siebkörpers 5 umgewälzt und mit neuer, aufnahmebereiter Flüssigkeit in Berührung gebracht, ohne daß sie mit den außerhalb des Siebkörpers 5 zurückgehaltenen Biokatalysatoren in Kontakt kommen.

Da es erfindungsgemäß wesentlich ist, daß die Sieböffnungen des Siebkörpers 5 eine relativ zur Teilchengröße der zurückzuhaltenden Teilchen deutlich größere freie Durchgangsweite aufweisen und somit die Rückhaltewirkung entscheidend durch die eingestellte Frequenz und Amplitude der Vibration bestimmt wird, kann deren Rückhaltung jederzeit durch einfaches Abstellen der Vibration oder durch geeignete Verringerung ihrer Frequenz und/oder Amplitude aufgehoben oder auf jeden gewünschten Wert begrenzt werden.

### Beispiel 1

In einem der in Fig. 1 dargestellten Vorrichtung ähnlichen Vibro-Fermenter (CHEMAP LF) mit einem etwa 30 cm hohen zylindrischen Behälter (Arbeitsvolumen 7 l) mit gewölbtem Boden und einem Bodenrührer (Durchmesser 10 cm) wurde eine mit einem Vibromischer verbundene Rückhaltevorrichtung mit einem unterseitig geschlossenen, vertikalen zylindrischen Siebkörper (Durchmesser 3 cm, Höhe 18 cm; Sieb aus rostfreiem Stahl, Quadratmaschengewebe mit Sieböffnungen 80 $\mu$m) zentrisch angeordnet. Der Siebkörper war durch vier Lochscheiben (Abstand 5 cm) mit einem am Vibromischer befestigten, durch eine zentrische Öffnung in den Behälter hineinragenden Gaseinleitrohr zu gemeinsamer Vibrationsbewegung verbunden.

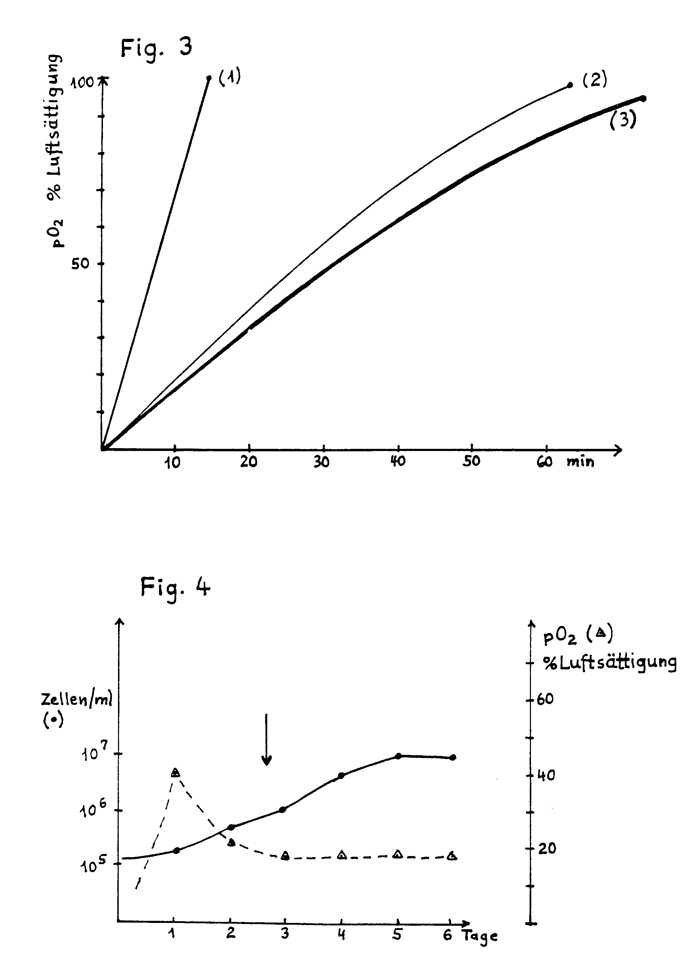

Der Behälter wurde mit 7 l Wasser befüllt und dessen Temperatur auf 37 °C eingestellt. Eine in den Behälter hineinragende $pO_2$-Sonde wurde so geeicht, daß bei einer Rührgeschwindigkeit von 30 U/min und Sättigung mit Luft auf der Sauerstoffskala ein Wert von 100 % angezeigt wurde. Zur Ermittlung der Belüftungsleistung wurde zunächst der Sauerstoffpartialdruck durch Begasen mit Stickstoff unter Vibration des Siebkörpers gegen 0 % abgesenkt und dann unter verschiedenen Betriebsbedingungen über den Innenraum des Siebkörpers jeweils so mit Luft bzw. mit reinem Sauerstoff begast, daß die Gasphase nur in dem vom Siebkörper umschlossenen Innenraum dispergiert wird und das Gas nur in gelöster Form durch das Sieb hindurch in den umgebenden Behälterraum gelangt. Die Ergebnisse dieser Versuch sind in Fig. 3 dargestellt. Die Kurve 1 wurde bei Einleiten von 7 l/h $O_2$ mit einer Amplitude von etwa 2 mm und die Kurven 2 und 3 bei Einleitung von 7 l/h Luft bei einer Amplitude von etwa 2 mm bzw. etwa 0,8 mm erhalten.

### Beispiel 2

Der gemäß Beispiel 1 verwendete Vibro-Fermenter war mit einer Sauerstoff-Regelvorrichtung versehen, die jeweils nach Erreichen eines vorbestimmten Sauerstoff-Partialdruckes die Gaszufuhr selbsttätig abschaltet und zusätzlich die Amplitude des Vibromischers auf einen einstellbaren Minimalwert herabsetzt. Sobald durch Sauerstoffverbrauch der vorbestimmte Sauerstoff-Partialdruck unterschritten wurde, öffnete die Regelvorrichtung automatisch die Gaszufuhr (Sauerstoff oder Luft) und setzte die Amplitude des Vibromischers wieder auf den höheren Wert herauf. Die zur Intensivierung der Belüftung dienende höhere Amplitude und die lediglich zur Freihaltung der Siebfläche von Teilchen beitragende niedrigere Amplitude konnten jeweils frei vorgewählt werden.

In dem so ausgestatteten Vibro-Fermenter wurden in einem Kultivationsversuch Säugetierzellen auf einem Microcarrier (Cytodex 3 der Firma Pharmacia, Uppsala) kultiviert. Die Konditionierung der Microcarrier, die Vorbehandlung des Zellinokulums und die Zellzählung erfolgten nach von der Firma Pharmacia veröffentlichten Standardmethoden. Während des Versuchs wurde im Fermenter eine Temperatur von 37° C und durch $CO_2$-Zugabe in das Belüftungsgas ein pH-Wert von 7,2 eingehalten. Die Rührer-Drehzahl betrug 30 U/min und der Sauerstoffpartialdruck entsprach 20% Luftsättigung ($pO_2$-Setpoint). Der Vibromischer war auf eine höhere Amplitude von etwa 1,2 mm und eine niedrigere Amplitude von etwa 0,5 mm eingestellt. Zur Belüftung wurden in den Kopfraum des Behälters pro Minute 200 ml Luft mit über den pH-Regler automatisch zugemischter $CO_2$-Menge und über den Siebkörper je nach automatischer Regelung über die Sauerstoff-Regelvorrichtung pro Minute 100 ml Sauerstoff eingeleitet.

Zur Bereitung des Inokulums wurden Zellen der Zellinie Mouse L in Rollerflaschen in DMEM (GIBCO) + 10% foetales Kälberserum (FCS) vorgezogen, nach 5 Tagen nach etablierten Versuchsprotokollen durch Trypsinisierung abgelöst und zu vorsterilisierten, nach etablierten Protokollen der Firma Pharmacia, Uppsala, in DMEM konditionierten Microcarriern Cytodex 3 zugesetzt. Die Inokulumsdichte wurde nach Auszählung in einer Thomakammer (Haemocytometer) so gewählt, daß 35 g Microcarrier in 2,5 Liter Nährlösung (DMEM + 10% FCS) mit etwa 7-8 x $10^8$ Zellen vermischt und unmittelbar nach dem Vermischen in den vorsterilisierten Fermenter überführt wurden. Über eine Zeitspanne von 4 Stunden wurde zwecks Besiedelung der Microcarrier mit Zellen jeweils im 1-2 Minuten-Zyklus bei 30-40 U/min gerührt und danach etwa 30 Minuten der Rührer abgestellt. Diese Prozedur wurde achtmal wiederholt. Danach wurde der Rührer auf eine Drehzahl von 30 U/min eingestellt, der Behälter langsam (über ca. 2 Stunden) mit Nährlösung (DMEM + 10% FCS) auf ein Endvolumen von 7 Liter aufgefüllt, der Kopfraum mit Gasmischung (Luft + $CO_2$) begast und die Kultur nach Einstellung obiger Setpoints sich selbst überlassen. Je einmal täglich wurde eine Probe gezogen und die Zellzahl nach etablierten Methoden bestimmt. Zwei Tage nach Beginn der Kultur wurde über einen Zulauf im Deckel des Fermenters mit einer Dosierpumpe 3 Liter frische Nährlösung pro Tag zudosiert und Kulturüberstand über das Niveaurohr innerhalb des Siebkörpers gemeinsam mit Gasphase abgezogen. Die in Fig. 4 dargestellten Versuchsergebnisse zeigen, daß nach etwa zwei Tagen die Belüftungskapazität aus dem Kopfraum des Behälters nicht mehr ausreichend war. Die mit dem Pfeil markierte Stelle zeigt den Beginn der automatischen Belüftung über den Siebkörper an.

**Patentansprüche**

1. Verfahren zum Behandeln einer suspendierte biologisch aktive Teilchen, wie beispielsweise Biokatalysatoren, Zellkulturen, Mikroorganismen, Mikrocarrier, enthaltenden flüssigen Mischung, bei welchem man aus dieser Mischung Flüssigkeit unter Zurückhaltung von Teilchen vorbestimmter Mindestgröße ins Innere einer in Schwingungen versetzten, im wesentlichen zylindrischen Siebfläche durchtreten läßt, **dadurch gekennzeichnet,** daß man in die von der Siebfläche umschlossene Flüssigkeitssäule Gas einführt und daß man die Siebfläche mit für die zurückzuhaltenden Teilchen im Ruhezustand passierbaren Öffnungen in deren Durchtritt mindestens weitgehend verhindernde, parallel zur Siebfläche gerichtete Schwingungen versetzt.

2. Verfahren nach Anspruch 1, **dadurch gekennzeichnet,** daß man eine Siebfläche mit die Durchschnittsgröße der zurückzuhaltenden Teilchen um 10 bis 50% übersteigender Durchgangsweite der Öffnungen mit einer auf die Durchschnittsgeschwindigkeit der Flüssigkeit abgestimmten Frequenz zwischen 10 und 100 Hertz und einer zwischen dem 0,5- und 50- fachen der Teilchengröße der zurückzuhaltenden Teilchen liegenden, deren Durchtritt hinreichend unterbindenden Amplitude vibrieren läßt.

3. Verfahren nach einem der Ansprüche 1 oder 2, **dadurch gekennzeichnet,** daß man in der Flüssigkeit an der Austrittsseite und/oder der Eintrittsseite der Siebfläche durchmischungsfördernde Strömungen erzeugt.

4. Verfahren nach einem der Ansprüche 1 bis 3, **dadurch gekennzeichnet,** daß man die Gaszufuhr bzw. die Frequenz und/oder die Amplitude der Vibration so regelt, daß mit gelöstem Gas angereicherte Flüssigkeit durch die Siebfläche in die die zurückzuhaltenden Teilchen enthaltende Mischung diffundiert.

5. Vorrichtung zum Behandeln einer suspendierte biologisch aktive Teilchen, wie beispielsweise Biokatalysatoren, Zellkulturen, Mikroorganismen, Mikrocarrier, enthaltenden flüssigen Mischung, mit einem Behälter (3) zur Aufnahme der Mischung,
   - einem in diesem vorzugsweise bis nahe zu dessen Boden (3') herunterreichend angeordneten im wesentlichen zylindrischen Siebkörper (5) und einer Vorrichtung (11) zum Einführen von Gas sowie
   - einer Vorrichtung (1) zum Bewegen des Siebkörpers (5) und
   - gegebenenfalls Vorrichtungen (8; 9) zum Einführen von Nährstoffen bzw. zum Ablauf der durch den Siebkörper (5) hindurchgetretenen Flüssigkeit,
   - wobei der Siebkörper (5) an der Unterseite geschlossen ist, **dadurch gekennzeichnet,** daß der Siebkörper (5) Öffnungen mit die Teilchengröße der zurückzuhaltenden Teilchen (6) übersteigender freier Durchgangsweite aufweist und mit einer Vorrichtung (1) zur Erzeugung einer den Durchtritt der zurückzuhaltenden Teilchen (6) durch die Öffnungen des Siebkörpers (5) mindestens weitgehend verhindernder paralleler zur Siebfläche des Siebkörpers (5) gerichteten Schwingung verbunden ist, und daß ein Gaseinleitrohr (11) zum Einführen von Gas in den vom Siebkörper umschlossenen Innenraum vorgesehen ist, wobei die Siebfläche vertikal angeordnet ist und der Siebkörper (5) über das darin zentral bis nahe an dessen unteren Ende reichende Gaseinleitrohr (11) an der Schwingungsvorrichtung (1) hängend angebracht ist.

6. Vorrichtung nach Anspruch 5, **dadurch gekennzeichnet,** daß im Innenraum des zylindrischen Siebkörpers (5) Mischeinrichtungen (12) zur Erzeugung von die umgebende Flüssigkeit durchmischenden Strömungen angeordnet sind.

**7.** Vorrichtung nach Anspruch 6, **dadurch gekennzeichnet,** daß eine oder mehrere den zylindrischen Siebkörper (5) mit dem zentrischen Gaseinleitrohr (11) verbindende Lochscheiben (12) vorgesehen sind.

**8.** Vorrichtung nach Anspruch 7, **dadurch gekennzeichnet,** daß mindestens eine Lochscheibe (12) über den Siebkörper (5) hinaus radial auswärts vorsteht.

**9.** Vorrichtung nach Anspruch 7 oder 8, **dadurch gekennzeichnet,** daß die Lochscheiben (12) sich nach oben bzw. nach unten stetig verengende Durchgangsöffnungen (16 bzw. 15) aufweisen.

**10.** Vorrichtung nach einem der Ansprüche 7 bis 9, **dadurch gekennzeichnet,** daß die Lochscheiben (12) innerhalb des Siebkörpers (5) zusätzliche Rückströmöffnungen (21) aufweisen.

**11.** Vorrichtung nach einem der Ansprüche 5 bis 10, **dadurch gekennzeichnet,** daß ein vom obersten Füllniveau des Behälters (3) ausgehendes Ablaufrohr (9) vorgesehen ist.

**12.** Vorrichtung nach einem der Ansprüche 5 bis 11, **dadurch gekennzeichnet,** daß das Gaseinleitrohr (11) mit Umschaltventilen (10) versehen und zum Abziehen von Flüssigkeit einsetzbar ist.

## Claims

**1.** A process for treating a liquid mixture containing suspended, biologically active particles, such as for example biocatalysts, cell cultures, micro-organisms, micro-carriers, wherein liquid from this mixture is caused to pass into the interior of a substantially cylindrical screening surface which is set in vibrations, with retention of particles of a predetermined minimum size, characterised in that gas is introduced into the liquid column enclosed by the screening surface and that the screening surface, which comprises openings which in the rest state are passable by the particles which are to be retained, is set in vibrations which at least substantially prevent passage through said openings and which are directed in parallel to the screening surface.

**2.** A process as claimed in Claim 1, characterised in that a screening surface, the openings of which possess a passage width which exceeds by 10 to 50% the average size of the particles which are to be retained, is caused to vibrate with a frequency of between 10 and 100 Hertz adapted to the average speed of the liquid and with an amplitude which amounts to between 0.5 and 50 times the size of the particles which are to be retained and which adequately inhibits the passage of said particles.

**3.** A process as claimed in one of Claims 1 or 2, characterised in that flows which serve to promote the mixing process are produced in the liquid at the outlet end and/or the inlet end of the screening surface.

**4.** A process as claimed in one of Claims 1 to 3, characterised in that the gas supply and the frequency and/or amplitude of the vibration are regulated in such manner that liquid enriched with dissolved gas diffuses through the screening surface into the mixture containing the particles which are to be retained.

**5.** A device for treating a liquid mixture containing suspended, biologically active particles, such as for example biocatalysts, cell cultures, micro-organisms, micro-carriers, comprising a container (3) which serves to accommodate the mixture,
- a substantially cylindrical screening body (5) which is arranged in said container (3) and projects downwards preferably towards the region of its base (3'), and a device (11) for introducing gas and a device for moving the screening body (5),
- optionally devices (8, 9) for introducing nutrients and discharging the liquid which has passed through the screening body (5),
- where the screening body (5) is closed on its underside, characterised in that the screening body (5) comprises openings with a free passage width which exceeds the size of the particles (6) which are to be retained and is connected to a device (1) for generating a vibration which at least substantially prevents the particles (6) which are to be retained from passing through the openings of the screening body (5) and which is directed in parallel to the screening surface of the screening body (5), and that a gas inlet pipe (11) is provided for introducing gas into the interior zone surrounded by the screening body, where the screening surface is arranged perpendicularly and the screening body (5) is attached to the vibration device (1) via the gas inlet pipe (11) which extends centrally in said screening body (5) towards the vicinity of its lower end.

6. A device as claimed in Claim 5, characterised in that mixing devices (12), which serve to produce flows which mix the surrounding liquid, are arranged in the interior of the cylindrical screening body (5).

7. A device as claimed in Claim 6, characterised in that one or several apertured discs (12) are provided which serve to connect the cylindrical screening body (5) to the central gas inlet pipe (11).

8. A device as claimed in Claim 7, characterised in that at least one apertured disc (12) projects radially outwards beyond the screening body (5).

9. A device as claimed in Claim 7 or 8, characterised in that the apertured discs (12) comprise through-openings (16; 15) which taper continuously upwards and downwards respectively.

10. A device as claimed in one of Claims 7 to 9, characterised in that the apertured discs (12) inside the screening body (5) comprise additional return flow openings (21).

11. A device as claimed in one of Claims 5 to 10, characterised in that an outlet pipe (9) is provided which commences from the uppermost filling level of the container (3).

12. A device as claimed in one of Claims 5 to 11, characterised in that the gas inlet pipe (11) is provided with switch-over valves (10) and can be used for the withdrawal of liquid.

**Revendications**

1. Procédé de traitement d'un mélange liquide contenant en suspension des particules actives biologiques, comme par exemple des biocatalyseurs, des cultures cellulaires, des micro-organismes, des microsupports, dans lequel on fait traverser une surface tamisante essentiellement cylindrique mise en vibration par le liquide de ce mélange en retenant à l'intérieur des particules d'une taille minimale déterminée, caractérisé en ce qu'on introduit du gaz dans la colonne de liquide entourée par la surface tamisante et qu'on provoque des vibrations parallèlement à la surface tamisante qui empêchent le passage des particules à retenir par les orifices où elles pourraient passer quand la surface tamisante est au repos.

2. Procédé selon la revendication 1, caractérisé en ce qu'on fait vibrer une surface tamisante, dont le diamètre de passage des orifices dépasse de 10 à 50 % le diamètre des particules à retenir, avec une fréquence accordée à la vitesse moyenne du liquide, comprise entre 10 et 100 Hertz et une amplitude qui est de 0,5 à 50 fois la taille des particules à retenir, suffisante pour empêcher leur passage.

3. Procédé selon l'une des revendications 2, caractérisé en ce qu'on provoque dans le liquide sur la face de sortie et/ou la face d'entrée de la surface tamisante des écoulements qui favorisent le mélange.

4. Procédé selon l'une des revendications 1 à 3, caractérisé en ce qu'on règle l'introduction de gaz ou la fréquence et/ou l'amplitude de la vibration, de sorte que le liquide enrichi en gaz dissous diffuse par la surface tamisante dans le mélange contenant les particules à retenir.

5. Dispositif pour le traitement d'un mélange liquide contenant par exemple des biocatalyseurs, des cultures de cellule, des micro-organismes, des microsupports avec une cuve (3) pour recueillir le mélange,
   - avec un corps tamisant (5) essentiellement cylindrique disposé dans celle-ci descendant de préférence à proximité de son fond (3') et un dispositif (11) pour introduire un gaz ainsi qu'un dispositif (1) pour mettre en mouvement le corps tamisant (5), ainsi que
   - le cas échéant des dispositifs (8 ; 9) pour introduire des produits nutritifs ou pour prélever le liquide traversant le corps tamisant (5),
   - le corps tamisant (5) étant fermé à sa face inférieure, caractérisé en ce que le corps tamisant (5) présente des orifices avec un diamètre de passage libre dépassant le diamètre des particules à retenir (6) et,
   - avec un dispositif (1) solidaire destiné à produire une vibration dirigée parallèlement à la surface tamisante du corps tamisant (5) empêchant largement le passage des particules (6) à retenir dans les orifices du corps tamisant (5), et
   - un tube d'introduction de gaz (11) est prévu pour introduire le gaz dans la chambre intérieure entourée par le corps tamisant, la surface tamisante est disposée, verticalement et le corps tamisant (5) est suspendu au dispositif de vibration (1) par le tube (11) d'introduction de gaz qui lui est centré et parvient jusqu'à sa partie inférieure.

6. Dispositif selon la revendication 5, caractérisé en ce qu'on implante dans la chambre interne du corps tamisant cylindrique (5), des dispositifs de mélange (12) destinés à provoquer des courants qui mélangent le liquide environnant.

7. Dispositif selon la revendication 6, caractérisé en ce qu'on implante un ou plusieurs disques percés (12) qui relient le corps tamisant cylindrique (5) au tube central d'introduction de gaz (11).

8. Dispositif selon la revendication 7, caractérisé en ce qu'au moins un disque percé (12) dépasse radialement au-delà du corps tamisant (5).

9. Dispositif selon la revendication 7 ou 8, caractérisé en ce que les disques percés (12) présentent des orifices de passage (16 ou 15) allant toujours en se rétrécissant vers le haut ou vers le bas.

10. Dispositif selon l'une des revendications 7 à 9, caractérisé en ce que les disques percés (12) présentent à l'intérieur du corps tamisant (5) des orifices supplémentaires (21) d'écoulement en retour.

11. Dispositif selon l'une des revendications 5 à 10, caractérisé en ce qu'on prévoit un tube d'écoulement (9) partant du niveau supérieur de remplissage de la cuve (3).

12. Dispositif selon l'une des revendications 5 à 11, caractérisé en ce que le tube d'introduction de gaz (11) est muni de vanne d'inversion (10) et peut être utilisé pour prélever du liquide.

Fig. 1

Fig. 2

## Fig. 3

## Fig. 4